# EUROPEAN PATENT SPECIFICATION

(11) **EP 1 609 492 B1**
(45) Date of publication and mention of the grant of the patent: **08.12.2010**
(21) Application number: 04724354.8
(22) Date of filing: 30.03.2004
(51) Int. Cl.: A61L 27/40, A61L 27/54, A61L 27/48, A61L 27/50

(54) **MOLDED ELASTIN ARTICLE AND PROCESS FOR PRODUCING THE SAME**
GEFORMTER ELASTIN-ARTIKEL UND VERFAHREN ZU SEINER HERSTELLUNG
ARTICLE EN ELASTINE MOULE ET SON PROCEDE DE PRODUCTION

(30) Priority: 31.03.2003 JP 2003094398
(43) Date of publication of application: 28.12.2005
(73) Proprietor: TEIJIN LIMITED, Osaka-shi Osaka 541-0054 (JP)
(72) Inventor: MIYAMOTO, Keiichi, Mie University, Tsu-shi, Mie 514-0008 (JP); KITAZONO, Eiichi, Teijin Limited, Hino-shi, Tokyo 191-0065 (JP); MIYOSHI, Takanori, Teijin Limited, Iwakuni-shi, Yamaguchi 740-0014 (JP); KANEKO, Hiroaki, Teijin Limited, Hino-shi, Tokyo 191-0065 (JP); SUMI, Yoshihiko, Teijin Limited, Hino-shi, Tokyo 191-0065 (JP); HIRATA, Hitoshi, Mie University, Tsu-shi, Mie 514-0001 (JP)
(74) Representative: Hallybone, Huw George
(86) International application number: PCT/JP2004/004494
(87) International publication number: WO 2004/087232

(56) References cited:
- EP-A- 0 005 035
- EP-A- 1 216 718
- WO-A-00/35372
- WO-A-01/82992
- WO-A-02/096978
- WO-A1-02/096978
- JP-A- 7 136 242
- JP-A- 9 273 080
- JP-A- 2001 514 049

## Description

### Technical Field

The present invention relates to an elastin molded article reinforced by use of a fiber structure comprising aliphatic polyester fibers having an average fiber diameter of 0.05 to 50 µm as a supporting base material.

### Background Art

In recent years, as a cure for severely damaged or lost living tissues and internal organs, a regenerative medical technique for restoring the severely damaged or lost living tissues and internal organs to their original conditions by taking advantage of differentiation and proliferation capabilities of cells has been vigorously studied. Neurotization is an example of the technique, and it has been studied that a tube made of artificial material is applied to a nerve damaged portion of a patient whose nervous tissue has been severed to bridge the open ends of the severed portion so as to guide the nervous tissue. As the tube, a tube which is made of silicon, polyurethane, polylactic acid, polyglycolic acid, polycaprolactone, a copolymer thereof or a mixture thereof and is internally coated with collagen or laminin is used.

Further, for revascularization, an artificial material tube which is made of polyurethane, polytetrafluoroethylene, polyester, polylactic acid, polyglycolic acid, polycaprolactone, a copolymer thereof or a mixture thereof and is internally coated with gelatin, albumin, collagen or laminin is used.

JP-A 8-33661 (the term "JP-A" as used herein means an "unexamined published Japanese patent application") describes an artificial blood vessel prepared by coacervating water-soluble elastin on the surface of the lumen of an artificial blood vessel material made of synthetic resin and fixing it by a crosslinking agent either directly or after gelatin or collagen is applied to the surface of the lumen and fixed by a crosslinking agent.

Further, JP-A 9-173361 describes an artificial blood vessel prepared by coacervating water-soluble elastin on an albumin layer formed by applying albumin on the surface of the lumen of an artificial blood vessel material made of synthetic resin, heating it and optionally crosslinking it by a crosslinking agent, and fixing the elastin by a crosslinking agent. However, the above silicon, polyurethane, polytetrafluoroethylene and polyester have a problem of long-term stability due to lack of biological absorption property and a problem of pressing or harming regenerated nerves or blood vessels. Further, a copolymer or mixture of a polylactic acid, polyglycolic acid or polycaprolactone has a biological absorption property but has a problem in compressive strength and also has a problem of pressing regenerated nerves or blood vessels. Incidentally, a Young's modulus required for a tube or artificial blood vessel to be implanted inside a body is 1 × 10⁴ to 2 × 10⁶ Pa.

In view of the above materials, Miyamoto et al. has reported in International Publication No. 02/096978 crosslinked elastin which is excellent in biological absorption property and compressive strength and can acquire a function of time-releasing a cell growth factor when hybridized with the cell growth factor. However, the crosslinked elastin is difficult to suture at the time of operation since it has a problem in tear strength and has a problem that its use inside a body is limited.

Tear strength required for suture at the time of operation is 0.3 MPa or higher.

EP 1 216 718 A1 discloses reinforced foam implants for soft tissue repair and regeneration.

### Disclosure of the Invention

A primary object of the present invention is to provide an elastin molded article used as a raw material for a tube or artificial blood vessel to be implanted in a body that has a biological absorption property and has tear strength and flexibility which allow the tube or artificial blood vessel to endure suture at the time of operation or the like.

Another object of the present invention is to provide a method for producing the above molded article of the present invention.

Other objects and advantages of the present invention will become apparent from the following description.

According to the present invention, firstly, the above objects and advantages of the present invention are achieved by an elastin molded article which comprises a fiber structure in the form of a nonwoven fabric comprising aliphatic polyester fibers having an average fiber diameter of 0.05 to 50 µm as a supporting base material and crosslinked elastin.

According to the present invention, secondly, the above objects and advantages of the present invention are achieved by a method for producing an elastin molded article characterized in that crosslinked elastin is formed by impregnating a fiber structure in the form of a nonwoven fabric comprising aliphatic polyester fibers having an average fiber diameter of 0.05 to 50 µm with water-soluble elastin and at least one water-soluble crosslinking agent and by causing a crosslinking reaction, wherein the fiber structure is placed in a mold in advance and a water-soluble elastin solution is obtained by mixing the water-soluble elastin and the at least one water-soluble crosslinking agent, and the water-soluble elastin solution is poured into the mold.

As described above, according to the present invention, there is provided an elastin molded article having flexibility, a biological absorption property and tear strength which is practically suturable by using a fiber structure comprising aliphatic polyester fibers having an average fiber diameter of 0.05 to 50 µm as a supporting base material.

### Brief Description of the Drawings

Fig. 1 is a schematic diagram showing an apparatus used in an electrospinning method which discharges a spinning solution into an electrostatic field in Examples.
Fig. 2 is a schematic diagram showing another apparatus used in the electrospinning method in Examples.

### Best Mode for Carrying out the Invention

An example of a fiber structure used in the present invention is a structure having form retainability which comprises an aggregation of one or more fibers. The fiber may be a surface smooth fiber, a porous fiber or a hollow fiber, for example. The fiber structure is in the form of a nonwoven fabric.

A polymer compound constituting the fiber structure is an aliphatic polyester.

Illustrative examples of the aliphatic polyester include a polylactic acid, a polyglycolic acid, a lactic acid-glycolic acid copolymer, a polycaprolactone, a polybutylene succinate, a polyethylene succinate, and copolymers thereof. Of these, the polylactic acid, polyglycolic acid, lactic acid-glycolic acid copolymer and polycaprolactone are preferred, and the polylactic acid and polycaprolactone are particularly preferred.

The fiber structure in the present invention is formed by fibers having an average fiber diameter of 0.05 to 50 µm. When the average fiber diameter is smaller than 0. 05 µm, biodegradability is high, so that time required for degradation is too short disadvantageously. Meanwhile, when the average fiber diameter is larger than 50 µm, the fibers show low stretchability when formed into a tube or the like, and the expression of elasticity unique to elastin is apt to be inhibited disadvantageously. The average fiber diameter is more preferably 0.2 to 25 µm, much more preferably 0.2 to 20 µm, particularly preferably 0.3 to 10 µm. The fiber diameter is a circle equivalent diameter of a fiber cross section defined by a circumference.

Specific examples of a method for producing the fiber structure in the present invention include an electrospinning method, a spunbonding method, a melt-blowing method and a flash spinning method. Of these, the electrospinning method is preferred. The electrospinning method can be implemented in accordance with the method disclosed in U.S. Patent No. 1,975,504.

The electrospinning method is conducted in the following manner, for example. The fiber structure can be obtained by discharging a solution having an aliphatic polyester dissolved in a volatile solvent into an electrostatic field formed between electrodes from a nozzle, drawing the discharged solution thinly toward the electrodes, and collecting the formed fibrous materials. The fibrous materials refer to not only a fiber structure from which the solvent of the solution has already been removed by distillation but also a fiber structure which still contains the solvent of the solution. The electrodes used in the present invention may be formed of any metal, inorganic material or organic material as long as the resulting electrodes show electrical conductivity. Further, the electrodes may be electrodes having a thin film of a metal, inorganic material or organic material showing electrical conductivity on an insulating material. The electrostatic field in the present invention is formed between a pair or plurality of electrodes, and a high voltage may be applied to any of the electrodes. This includes, for example, a case where a total of three electrodes, i.e., two high-voltage electrodes having different voltage values, e.g., electrodes of 15 kV and 10 kV, and an earthed electrode, and a case where four or more electrodes are used.

The concentration of aliphatic polyester in the aliphatic polyester solution used in electrospinning is preferably 1 to 30 wt%. When the concentration of the aliphatic polyester is lower than 1 wt%, the concentration is so low that the fiber structure is difficult to form disadvantageously. Meanwhile, when the concentration is higher than 30 wt%, the fiber diameter of the fiber structure to be obtained becomes large disadvantageously. The concentration of the aliphatic polyester is more preferably 2 to 20 wt%.

The volatile solvent forming the aliphatic polyester solution used in electrospinning in the present invention is a substance which dissolves an aliphatic polyester and preferably has a boiling point at normal pressures of not higher than 200°C and is liquid at 27°C.

Specific examples of the volatile solvent include methylene chloride, chloroform, acetone, methanol, ethanol, propanol, isopropanol, toluene, tetrahydrofuran, 1,1,1,3,3,3-hexafluoroisopropanol, water, 1,4-dioxane, carbon tetrachloride, cyclohexane, cyclohexanone, N,N-dimethylformamide, and acetonitrile. Of these, methylene chloride, chloroform and acetone are particularly preferred in view of solubility of the aliphatic polyester.

These solvents may be used alone or in combination of two or more. Further, other solvents may be used in combination with the above solvents as long as the object of the present invention is not impaired.

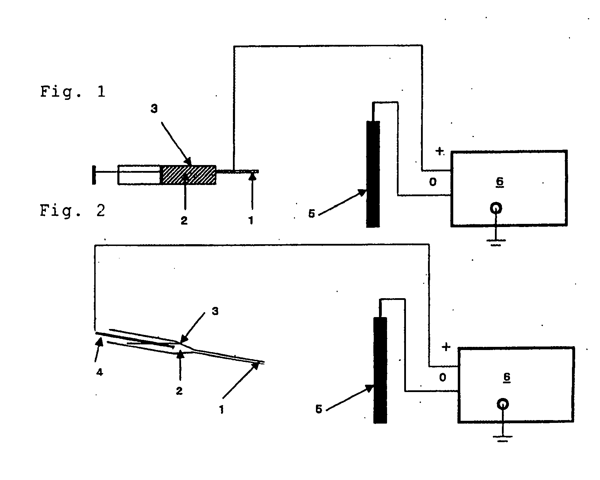

To discharge the solution into an electrostatic field, any method can be used. An example of the method will be described hereinafter by use of Fig. 1. A solution 2 of an aliphatic polyester in a volatile solvent is fed to a nozzle 1 to allocate the solution at an appropriate position in an electrostatic field, and the solution is drawn thinly from the nozzle by an electric field to form a fiber. Appropriate equipment can be used to form the fiber. For example, to the front end of a solution container 3 which has a cylindrical shape resembling a syringe body, appropriate means, e.g., an injection-needle-like solution discharge nozzle 1 to which a voltage has been applied by a high-voltage generator 6, is attached, and the solution is lead to the tip thereof. The tip of the discharge nozzle 1 is placed at a proper distance from an earthed fibrous material collecting electrode 5, and when the solution 2 is discharged from the tip of the discharge nozzle 1, a volatile solvent is evaporated between the tip thereof and the fibrous material collecting electrode 5 so as to form a fibrous material.

Further, those skilled in the art can introduce fine drops of the solution into the electrostatic field by a self-evident method. An example thereof will be described hereinafter by use of Fig. 2. The only requirement therefor is that the solution is placed in the electrostatic field and kept away from the fibrous material collecting electrode 5 at a distance which may cause formation of the fiber. To place the solution in the electrostatic field, an electrode 4 which opposes the fibrous material collecting electrode may be inserted into the solution 2 in the solution container 3 which has the nozzle 1 directly, for example.

When the solution is fed into the electrostatic field from the nozzle, the production rate of the fibrous material can be increased by use of a plurality of nozzles. As for the distance between the electrodes, although it depends on a charging level, the size of the nozzle, the flow rate of the spinning solution and the concentration of the spinning solution, a distance of 5 to 20 cm is appropriate for about 10 kV. Further, an electrostatic potential to be applied is 3 to 100 kV, preferably 5 to 50 kV, more preferably 5 to 30 kV, for example. A desired potential can be generated by any appropriate method.

The above description has been given to a case where the electrode also serves as both a collector and electrode. In other case, a collector independent of the electrodes may be disposed between the electrodes. Further, a sheet, a tube or the like can be obtained by selecting the shape of the collector. Further, continuous production becomes possible by disposing a belt-like material between the electrodes as a collector.

In the present invention, while the solution is drawn thinly toward the collector, the solvent is evaporated according to the condition to form the fibrous material. In general, at room temperature, the solvent is evaporated completely until the fibrous material is collected on the collector. In some cases, the solution may be drawn under reduced pressure conditions to fully evaporate the solvent. Further, the drawing temperature depends on the evaporation behavior of the solvent and the viscosity of the spinning solution. For example, the drawing temperature is 0 to 50°C. Thereby, the fibrous material is collected on the collector to produce the fiber structure.

The fiber structure obtained in the present invention may be used alone or used in combination with other members according to ease of handling and other requirements. For example, a composite member which is a combination of a supporting base material and the fibrous material can be prepared by using a nonwoven fabric, a woven fabric, a film or the like which can be the supporting base material as the collector and forming the fiber structure thereon.

Water-soluble elastin used in the present invention is not particularly limited. The water-soluble elastin is obtained by hydrolysis of elastin. More specifically, at least one elastin, e.g., α-elastin or β-elastin obtained by subjecting the ligament of the neck of an animal or the like to a thermal oxalic acid treatment, κ-elastin obtained by subjecting β-elastin or elastin to an alkali ethanol treatment, water-soluble elastin enzyme-treated with elastase, and tropoelastin which is a precursor in an elastin biosynthetic pathway, can be used. The tropoelastin is not particularly limited. An extract from an animal call or at least one tropoelastin gene product obtained by gene recombination can be used.

Crosslinked elastin in the present invention can be obtained by crosslinking at least one water-soluble elastin by a water-soluble crosslinking agent.

The water-soluble elastin is a hydrophobic protein in which a hydrophobic amino acid constitutes about 94% of the total weight and an amino acid having an amino group in a side chain, e.g., lysine, arginine or hystidine, constitutes about 1% of the total weight.

The water-soluble crosslinking agent used in the present invention may be any water-soluble crosslinking agent which reacts with the amino acid in the side chain of the water-soluble elastin and causes a crosslinking reaction. Illustrative examples of the water-soluble crosslinking agent include glutaraldehyde, ethylene glycidyl ether, and a compound represented by the following formula which has a hydrophobic portion in the central region of the molecule and has an active ester group at both ends of the molecule. In particular, when the compound represented by the following formula (1) is used as a crosslinking agent, a molded article having elasticity suitable for a living body and good moldability can be preferably obtained. 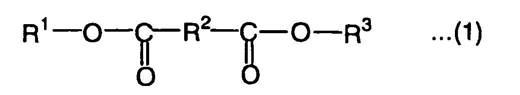 wherein R¹ and R³ each independently represent a structure represented by the following formula (1)-1: 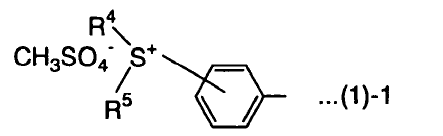 wherein R⁴ and R⁵ each independently represent H, CH₃ or C₂H₅,
or a structure represented by the following formula (1)-2: 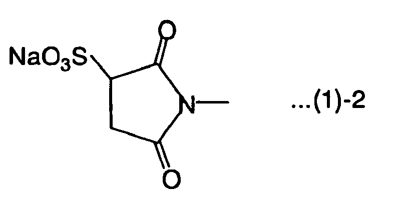 and, R² represents a structure represented by the following formula (1)-3: 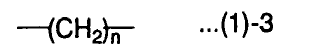 wherein n is 1 to 20,
or a structure represented by the following formula (1)-4: 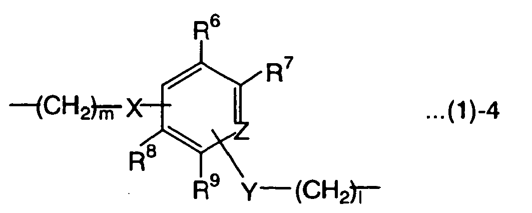 wherein m and 1 each independently represent an integer of 0 to 15, X and Y each independently represent CH₂ or O, Z represents C or N, and R⁶, R⁷, R⁸ and R⁹ each independently represent H, CH₃ or C₂H₅.

Together with elastin containing a large quantity of hydrophobic amino acid, the compound having a hydrophobic portion in the central region of the molecule forms a strong and stable structure by hydrophobic interaction.

However, since a compound containing a large quantity of hydrophobic portion is soluble in an organic solvent but is hardly soluble or insoluble in water, it is difficult to handle the compound in a water system. The water-soluble crosslinking agent can be produced by, for example, active-esterifying both ends of a corresponding dicarboxylic acid compound by use of 4-hydroxyphenyldimethyl-sulfonium methyl sulfate (hereinafter referred to as "DSP"). This water-soluble crosslinking agent is characterized in that it has a hydrophobic portion which forms a strong and stable structure together with elastin containing a large quantity of hydrophobic amino acid and that it can be handled in the water system.

Further, in the present invention, the water-soluble crosslinking agent achieves crosslinking through peptide bonds between the active ester groups at both ends of the chemical formula of the water-soluble crosslinking agent and the amino acids in the water-soluble elastin. Conditions for the crosslinking reaction are not particularly limited. The reaction temperature preferably ranges from 4° C to 150° C at normal pressures or under pressure applied by an autoclave. The reaction temperature particularly preferably ranges from 10°C to 120°C in view of operability in crosslinking.

In the present invention, the crosslinked elastin may contain a third component, in addition to the water-soluble elastin and the crosslinking agent.

Illustrative examples of the third component include proteins such as collagen, gelatin, fibronectin, fibrin, laminin, casein, keratin, sericin and thrombin, polyamino acids such as a polyasparatic acid, a polyglutamic acid and a polylysine, sugars such as a polygalacturonic acid, heparin, chondroitin sulfuric acid, hyaluronic acid, dermatan sulfuric acid, chondroitin, dextran sulfuric acid, sulfated cellulose, alginic acid, dextran, carboxymethyl chitin, galactomannan, gum acacia, tragacanth gum, gelin gum, sulfated gelin, karaya gum, carrageenan, agar, xanthan gum, curdlan, pullulan, cellulose, starch, carboxymethyl cellulose, methyl cellulose, glucomannan, chitin, chitosan, xyloglucan and lentinan, and/or cell growth factors such as FGF (fibroblast growth factor), EGF (epidermal growth factor), PDGF (platelet-derived growth factor), IGF (insulin-like growth factor), VEGF (vascular endothelial growth factor), TGF-β (β-type transforming growth factor), NGF (nerve growth factor), HGF (hepatocellular growth factor), and BMP (bone morphogenetic factor).

Of these, extracellular matrix components such as gelatin, collagen, fibronectin, laminin, heparin and chondroitin sulfuric acid and cell growth factors such as FGF (fibroblast growth factor), EGF (epidermal growth factor), VEGF (vascular endothelial growth factor), NGF (nerve growth factor) and HGF (hepatocellular growth factor) are preferred because they enhance bonding and proliferation of cells.

In the present invention, the proportion of the water-soluble elastin is preferably 0.5 to 99.5 wt% based on the crosslinked elastin. The proportion is more preferably 1 to 95 wt%. With the proportion thereof within this range, a molded article having elasticity suitable for a living body and good moldability can be obtained.

In the present invention, a method for producing an elastin molded article reinforced by the fiber structure in the form of a nonwoven fabric comprises

using a mold used to mold a synthetic resin. When a fiber structure is placed in a mold in advance and a water-soluble elastin solution is obtained by mixing water-soluble elastin and a water-soluble crosslinking agent together, poured into the mold and heated by an autoclave or the like to cause a crosslinking reaction, a film-, stick-, pellet- or tube-shaped elastin molded article reflecting the shape of the mold can be obtained.

In the present invention, the crosslinked elastin obtained by crosslinking by the water-soluble crosslinking agent has a characteristic that it is susceptible to biodegradation in a living body. Since the speed of the biodegradation relates to the degree of crosslinking of the crosslinked elastin, it can be controlled by changing the degree of crosslinking by changing conditions for crosslinking.

The crosslinked elastin in the present invention is a crosslinked protein having excellent elasticity. To be adapted to a living body easily, the crosslinked elastin preferably has a Young's modulus of 1 × 10² to 1 × 10⁷ Pa, particularly preferably 1 × 10³ to 2 × 10⁶ Pa.

As described above, according to the present invention, the elasticity and flexibility of elastin can be retained and tear strength which allows suture can be imparted to crosslinked elastin by use of a fiber structure comprising various aliphatic polyester fibers such as hollow fibers or porous fibers having an average particle diameter of 0.05 to 50 µm as a supporting base material. Such an elastin molded article is useful as an artificial material in revascularization and neurotization.

### Examples

Details of the present invention will be further described by use of the following Examples. However, the present invention shall not be limited by these Examples in any way.

In these Examples, a polylactic acid (LACTY9031) of Shimadzu Corporation, elastin of ELASTIN PRODUCTS CO., LTD., and methylene chloride (special grade), oxalic acid (special grade), a cellulosic dialysis tube (molecular weight cut off: 6,000 to 10,000), dodecanedicarboxylic acid, 4-hydroxyphenyldimethyl-sulfonium methyl sulfate, dicyclohexyl carbodiimide, acetonitrile (special grade) and triethylamine of Wako Pure Chemical Industries, Ltd. were used.

### Preparation Method of Polylactic Acid Tube

1 g of polylactic acid and 8 g of methylene chloride were mixed together at room temperature (25°C) to prepare a dope. The dope was discharged to a fibrous material collecting electrode 5 (diameter: 2 mm, length: 200 mm) which spun at 60 rpm, for 5 minutes by use of the apparatus shown in Fig. 2. The internal diameter of a discharge nozzle 1 was 0.8 mm, the voltage was 12 kV, and the distance from the discharge nozzle 1 to the fibrous material collecting electrode 5 was 10 cm. The obtained polylactic acid tube had an internal diameter of 2 mm and a length of 20 mm. Further, the coating amount was controlled by varying the discharge time, and two types of samples one of which had a coating amount of 20 g/m² and the other of which had a coating amount of 40 g/m² were prepared.

### Preparation Method of Water-Soluble Elastin

150 ml of 0.25M oxalic acid was added to 20 g of elastin (product of ELASTIN PRODUCTS CO., LTD.) and treated at 100° C for 1 hour. After cooled, the mixture was centrifuged (3,000 rpm, 30 minutes), the supernatant liquid was collected and charged into a cellulosic dialysis tube, and oxalic acid was removed by performing dialysis with deionized water for 48 hours. Then, the resulting product was freeze-dried to give water-soluble elastin.

### Preparation Method of Water-Soluble Crosslinking Agent

0.64 g (2.5 mmol) of dodecanedicarboxylic acid and 1.33 g (5 mmol) of 4-hydroxyphenyldimethyl-sulfonium methyl sulfate were dissolved into 35 ml of acetonitrile at 60°C. After the mixture was left to stand to be cooled, 1.03 g (5 mmol) of dicyclohexyl carbodiimide was added, and the resulting mixture was agitated at 25°C for 5 hours. Then, dicyclohexyl urea produced during the reaction was removed through filtration using a glass filter. Further, the filtrate was dropped to 70 ml of ether to be solidified. The solid was vacuum-dried to give 1.4 g of water-soluble crosslinking agent. The purity of the obtained crosslinking agent was found to be 98% by ¹H-NMR.

### Example 1

200 mg of the water-soluble elastin was added to 1 ml of deionized water and agitated to give a 20% water-soluble elastin solution. The temperature of the solution was adjusted to 25°C. To the solution, 72 µmol (three times as large as the amount (24 µmol) of amino groups in the elastin in the solution) of the water-soluble crosslinking agent was added and agitated for 5 minutes. Then, 24 µmol of triethylamine was added, and the mixture was agitated for another 5 minutes. Then, the mixture was poured into a cylindrical template having a diameter of 2.2 mm and a length of 30 mm on which the polylactic acid tube (coating amount: 20 g/m²) was mounted and then left to stand for two days to be gelled. The obtained gel was fully rinsed with deionized water to obtain a milky-white cylindrical elastin molded article having excellent elasticity. Further, the obtained elastin molded article was treated with an autoclave at 110°C for 10 minutes to obtain a sterilized elastin molded article whose shape remained unchanged. The Young's modulus of the obtained elastin molded article was 1 × 10⁵ Pa.

The tear strength of the obtained molded article was measured by use of TENSILON (INSTRON) with reference to DIN53507 and DIN53504. The result is shown in Table 1.

### Example 2

The procedure of Example 1 was repeated except that the coating amount of the polylactic acid tube was 40 g/m². The Young's modulus of the obtained elastin molded article was 1 × 10⁶ Pa.

### Comparative Example 1

The tear strength of elastin prepared with reference to Example 1 was measured.

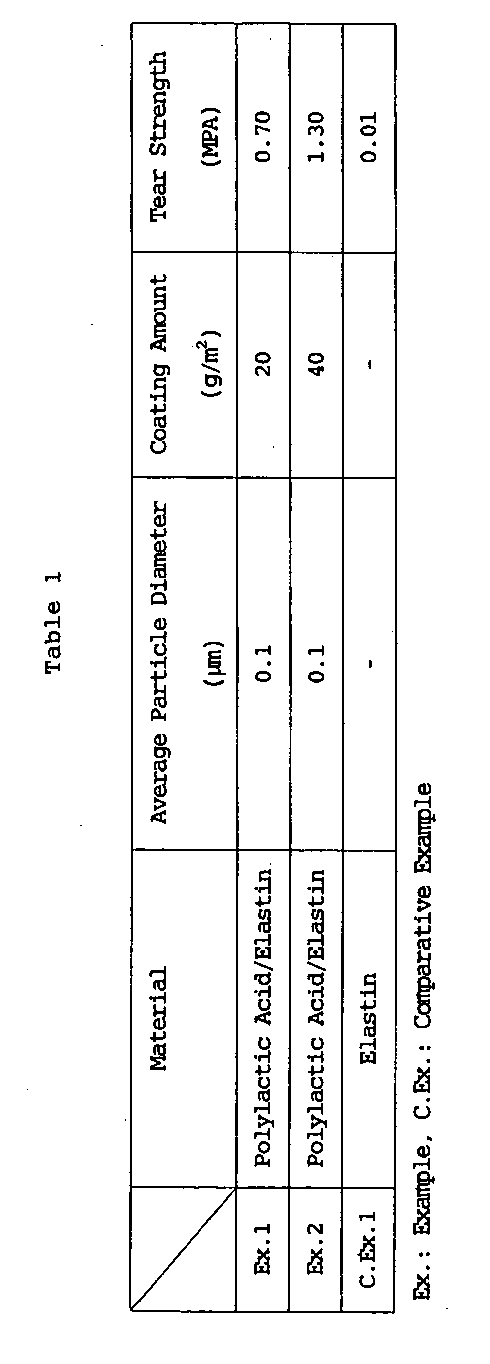

## Claims

1. An elastin molded article which comprises a fiber structure in the form of a nonwoven fabric comprising aliphatic polyester fibers having an average fiber diameter of 0.05 to 50 µm as a supporting base material and crosslinked elastin.

2. The elastin molded article according to claim 1, wherein the aliphatic polyester is a polylactic acid, a polyglycolic acid, a polycaprolactone or a copolymer thereof.

3. The elastin molded article according to claim 1, wherein the fiber is a surface smooth fiber, a porous fiber or a hollow fiber.

4. The elastin molded article according to claim 1, wherein the crosslinked elastin comprises a product resulting from a reaction of water-soluble elastin with at least one crosslinking agent.

5. The elastin molded article according to claim 4, wherein the crosslinking agent is a water-soluble compound represented by the following formula (1): 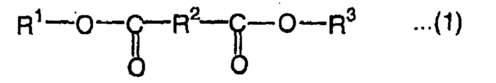 wherein R¹ and R³ each independently represent a structure represented by the following formula (1)-1: 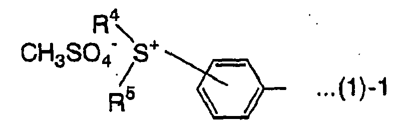 wherein R⁴ and R⁵ each independently represent H, CH₃ or C₂H₅,
or a structure represented by the following formula (1)-2: 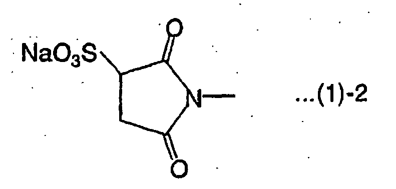 and, R² represents a structure represented by the following formula (1)-3: 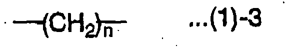 wherein n is 1 to 20,
or a structure represented by the following formula (1)-4: 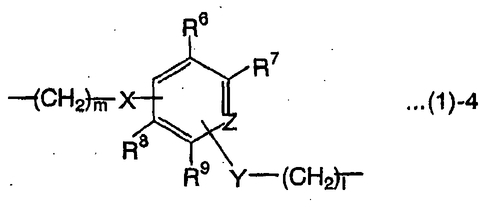 wherein m and 1 each independently represent an integer of 0 to 15, X and Y each independently represent CH₂ or O, Z represents C or N, and R⁶, R⁷, R⁸ and R⁹ each independently represent H, CH₃ or C₂H₅.

6. The elastin molded article according to claim 1, wherein the crosslinked elastin further contains at least one selected from the group consisting of a protein, a polyamino acid, sugar and a cell growth factor.

7. The elastin molded article according to claim 6, wherein the protein is collagen, gelatin, fibronectin, fibrin, thrombin or laminin.

8. The elastin molded article according to claim 6, wherein the polyamino acid is a polylysine or a polyglutamic acid.

9. The elastin molded article according to claim 6, wherein the sugar is hyaluronic acid, chondroitin sulfuric acid, heparin, alginic acid, chitin, chitosan, cellulose or starch

10. The elastin molded article according to claim 6, wherein the cell growth factor is FGF (fibroblast growth factor), EGF (epidermal growth factor), PDGF (platelet-derived growth factor), IGF (insulin-like growth factor), VEGF (vascular endothelial growth factor) , TGF-β (β-type transforming growth factor) , NGF (nerve growth factor), HGF (hepatocellular growth factor) or BMP (bone morphogenetic factor).

11. A method for producing an elastin molded article **characterized in that** crosslinked elastin is formed by impregnating a fiber structure in the form of a nonwoven fabric comprising aliphatic polyester fibers having an average fiber diameter of 0.05 to 50 µm with water-soluble elastin and at least one water-soluble crosslinking agent and by causing a crosslinking reaction, wherein the fiber structure is placed in a mold in advance and a water-soluble elastin solution is obtained by mixing the water-soluble elastin and the at least one water-soluble cross-linking agent, and the water-soluble elastin solution is poured into the mold.

12. The method according to claim 11, wherein the fiber is a surface smooth fiber, a porous fiber or a hollow fiber.

## Patentansprüche

1. Elastin-geformter Gegenstand, welcher eine Faserstruktur in der Form eines Faservlieses umfaßt, welche aliphatische Polyesterfasern mit einem durchschnittlichen Faserdurchmesser von 0,05 bis 50 µm als ein stützendes Basismaterial und vernetztes Elastin umfaßt.

2. Elastin-geformter Gegenstand gemäß Anspruch 1, wobei der aliphatische Polyester eine Polymilchsäure, eine Polyglycolsäure, ein Polycaprolacton oder ein Copolymer davon ist.

3. Elastin-geformter Gegenstand gemäß Anspruch 1, wobei die Faser eine oberflächenglatte Faser, eine poröse Faser oder eine Hohlfaser ist.

4. Elastin-geformter Gegenstand gemäß Anspruch 1, wobei das vernetzte Elastin ein Produkt umfaßt, resultierend aus einer Reaktion von wasserlöslichem Elastin mit mindestens einem Vernetzungsmittel.

5. Elastin-geformter Gegenstand gemäß Anspruch 4, wobei das Vernetzungsmittel eine wasserlösliche Verbindung, dargestellt durch die folgende Formel (1) ist: 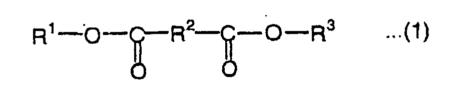 worin R¹ und R³ jeweils unabhängig voneinander eine Struktur, dargestellt durch die folgende Formel (1)-1 : 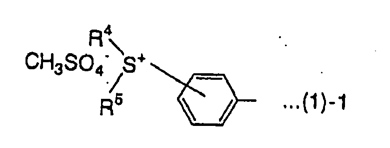 worin R⁴ und R⁵ jeweils unabhängig voneinander H, CH₃ oder C₂H₅ darstellen, oder eine Struktur darstellen, dargestellt durch die folgende Formel (1) - 2: 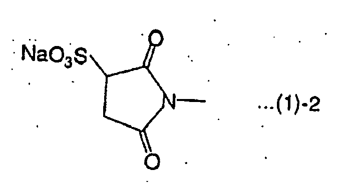 und R² eine Struktur, dargestellt durch die folgende Formel (1) - 3: 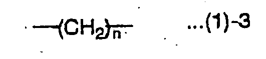 worin n 1 bis 20 ist oder eine Struktur darstellt, dargestellt durch die folgende Formel (1) - 4: 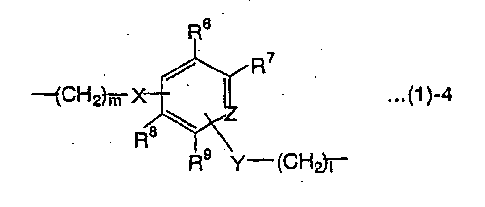 worin m und I jeweils unabhängig voneinander eine ganze Zahl von 0 bis 15 darstellen, X und Y jeweils unabhängig voneinander CH₂ oder O darstellen, Z C oder N darstellt und R⁶, R⁷, R⁸ und R⁹ jeweils unabhängig voneinander H, CH₃ oder C₂H₅ darstellen.

6. Elastin-geformter Gegenstand gemäß Anspruch 1, wobei das vernetzte Elastin weiter mindestens eines enthält, ausgewählt aus der Gruppe, bestehend aus einem Protein, einer Polyaminosäure, Zucker oder einem Zellwachstumsfaktor.

7. Elastin-geformter Gegenstand gemäß Anspruch 6, wobei das Protein Kollagen, Gelatin, Fibronectin, Fibrin, Thrombin oder Laminin ist.

8. Elastin-geformter Gegenstand gemäß Anspruch 6, wobei die Polyaminosäure ein Polylysin oder eine Polyglutaminsäure ist.

9. Elastin-geformter Gegenstand gemäß Anspruch 6, wobei der Zucker Hyaluronsäure, Chondroitinsschwefelsäure, Heparin, Algininsäure, Chitin, Chitosan, Cellulose oder Stärke ist.

10. Elastin-geformter Gegenstand gemäß Anspruch 6, wobei der Zellwachstumsfaktor FGF (Fibroblastwachstumsfaktor), EGF (epidermaler Wachstumsfaktor), PDGF (Plättchen-abgeleiteter Wachstumsfaktor), IGF (Insulin-ähnlicher Wachstumsfaktor), VEGF (vaskulärer endothelialer Wachstumsfaktor), TGF-β (β-Typ-Umwandlungswachstumsfaktor), NGF (Nervenwachstumsfaktor), HGF (hepatozellulärer Wachstumsfaktor) oder BMP (knochenmorphogenetischer Faktor) ist.

11. Verfahren zur Herstellung eines Elastin-geformten Gegenstands, **gekennzeichnet dadurch, daß** vernetztes Elastin durch Imprägnieren einer Faserstruktur in der Form eines Faservlieses, umfassend aliphatische Polyesterfasern mit einem durchschnittlichen Faserdurchmesser von 0,05 bis 50 µm, mit wasserlöslichem Elastin und mindestens einem wasserlöslichen Vernetzungsmittel und durch Bewirken einer Vernetzungsreaktion gebildet wird, wobei die Faserstruktur zuvor in einer Form angeordnet wird und eine wasserlösliche Elastinlösung durch Mischen des wasserlöslichen Elastins und des mindestens einen wasserlöslichen Vernetzungsmittels erhalten wird und die wasserlösliche Elastinlösung in die Form gefüllt wird.

12. Verfahren gemäß Anspruch 11, wobei die Faser eine oberflächenglatte Faser, eine poröse Faser oder eine Hohlfaser ist.

## Revendications

1. Article moulé en élastine, lequel comprend une structure fibreuse sous forme d'un tissu non tissé comprenant des fibres de polyester aliphatique ayant un diamètre de fibre moyen de 0,05 à 50 µm en tant que matériau de base support et de l'élastine réticulée.

2. Article moulé en élastine selon la revendication 1, dans lequel le polyester aliphatique est un acide polylactique, un acide polyglycolique, une polycaprolactone ou un copolymère de ceux-ci.

3. Article moulé en élastine selon la revendication 1, dans lequel la fibre est une fibre à surface lisse, une fibre poreuse ou une fibre creuse.

4. Article moulé en élastine selon la revendication 1, dans lequel l'élastine réticulée comprend un produit résultant d'une réaction de l'élastine soluble dans l'eau avec au moins un agent de réticulation.

5. Article moulé en élastine selon la revendication 4, dans lequel l'agent de réticulation est un composé soluble dans l'eau représenté par la formule suivante (1) : 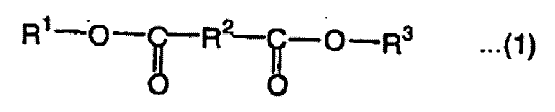 dans laquelle R¹ et R³ représentent chacun indépendamment une structure représentée par la formule suivante (1)-1 : 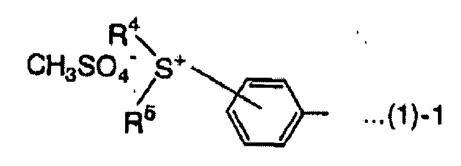 dans laquelle R⁴ et R⁵ représentent chacun indépendamment H, CH₃ ou C₂H₅,
ou une structure représentée par la formule suivante (1)-2 : 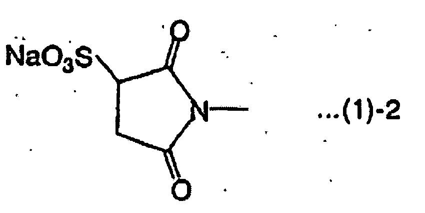 et R² représente une structure représentée par la formule suivante (1)-3 : 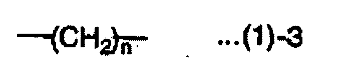 dans laquelle n vaut entre 1 et 20,
ou une structure représentée par la formule suivante (1)-4 : 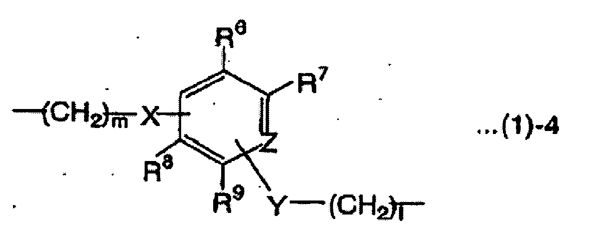 dans laquelle m et 1 représentent chacun indépendamment un nombre entier entre 0 et 15, X et Y représentent chacun indépendamment CH₂ ou O, Z représente C ou N, et R⁶, R⁷, R⁸ et R⁹ représentent chacun indépendamment H, CH₃ ou C₂H₅.

6. Article moulé en élastine selon la revendication 1, dans lequel l'élastine réticulée contient en outre au moins un élément choisi dans le groupe constitué par une protéine, un acide polyaminé, un sucre et un facteur de croissance cellulaire.

7. Article moulé en élastine selon la revendication 6, dans lequel la protéine est le collagène, la gélatine, la fibronectine, la fibrine, la thrombine ou la laminine.

8. Article moulé en élastine selon la revendication 6, dans lequel l'acide polyaminé est une polylysine ou un acide polyglutamique.

9. Article moulé en élastine selon la revendication 6, dans lequel le sucre est l'acide hyaluronique, l'acide chondroïtine sulfurique, l'héparine, l'acide alginique, la chitine, le chitosane, la cellulose ou l'amidon.

10. Article moulé en élastine selon la revendication 6, dans lequel le facteur de croissance cellulaire est le FGF (facteur de croissance des fibroblastes), l'EGF (facteur de croissance épidermique), le PDGF (facteur de croissance dérivé des plaquettes), l'IGF (facteur de croissance insulinomimétique), le VEGF (facteur de croissance endothélial vasculaire), le TGF-β (facteur de croissance transformant β), le NGF (facteur de croissance du tissu nerveux), le HGF (facteur de croissance hépatocellulaire) ou le BMP (facteur morphogénétique osseux).

11. Procédé de production d'un article moulé en élastine **caractérisé en ce que** l'élastine réticulée est formée en imprégnant une structure fibreuse sous forme d'un tissu non tissé comprenant des fibres de polyester aliphatique ayant un diamètre de fibre moyen de 0,05 à 50 µm avec de l'élastine soluble dans l'eau et au moins un agent de réticulation soluble dans l'eau et en provoquant une réaction de réticulation, dans lequel la structure fibreuse est placée dans un moule à l'avance et une solution d'élastine soluble dans l'eau est obtenue en mélangeant l'élastine soluble dans l'eau et ledit au moins un agent de réticulation soluble dans l'eau, puis la solution d'élastine soluble dans l'eau est versée dans le moule.

12. Procédé selon la revendication 11, dans lequel la fibre est une fibre à surface lisse, une fibre poreuse ou une fibre creuse.
